# EUROPEAN PATENT SPECIFICATION

(11) **EP 1 633 201 B1**
(45) Date of publication and mention of the grant of the patent: **05.10.2011**
(21) Application number: 04722545.3
(22) Date of filing: 23.03.2004
(51) Int. Cl.: A23K 1/18, A23K 1/16, A23K 1/175

(54) **PET FOOD COMPOSITION**
HAUSTIER-FUTTERMITTELZUSAMMENSETZUNG
COMPOSITION D'ALIMENT POUR ANIMAUX FAMILIERS

(30) Priority: 16.06.2003 EP 03013622
(43) Date of publication of application: 15.03.2006
(73) Proprietor: DSM IP Assets B.V., 6411 TE Heerlen (NL)
(72) Inventor: KRAMMER, Stephanie, 79541 Loerrach-Hauingen (DE); PHEIFFER, Joachim, 79539 Loerrach (DE)
(74) Representative: Schwander, Kuno
(86) International application number: PCT/EP2004/003062
(87) International publication number: WO 2004/110164

(56) References cited:
- EP-A- 0 848 955
- WO-A-02/058662
- US-B1- 6 299 912
- OXBOW PET PRODUCTS - PRODUCT DETAILS FOR CAVY CUISINE, [Online] 24 May 2002 (2002-05-24), - 24 May 2002 (2002-05-24) XP002284498 Retrieved from the Internet: URL:http://web.archive.org/web/20021019222 802/www.oxbowhay.com/showProduct.sp?PRODUC T_NO=47&cat=46> [retrieved on 2004-06-18]

## Description

The present invention relates to pet food compositions which are useful for preventing or treating calculus, plaque, gingivitis and periodontal disease in pets.

From oxbow pet products - product details for cavy cuisine,
XP 002284498 (Retrieved from the Internet: URL:http://web.archive. org/web/20021019222802/www.oxybowhay.com/showProduct.sp?PRODUC T_NO=47&cat=46> a pet food formulation is known which comprises at minimum 0.04% by weight of Stay C-35 (sodium-calcium L-ascorbic acid-2-monophosphate).
US 6'299'912 discloses a pet feed composition which comprises less than 1% of sodium calcium ascorbic acid phosphate. EP 0'848'955 discloses a pet feed composition which comprises 0.03% of trisodium ascorbyl monophosphate and WO 02/058662 discloses the use of the trisoldium salt of ascorbyl-2-monophosphate in the preparation of composition for the treatment of dental problems.

Other than that mentioned background art, the present invention relates, to trisodium L-ascorbic- acid-2-monophosphate for use in the manufacture of a pet food, particularly for preventing or treating calculus, plaque, gingivitis and periodontal disease in pets.

The term "pet" as used herein includes dogs, cats and rodents, e.g., chinchillas, guinea pigs, degus, mice, gerbils, hamsters, rats, ferrets and lagomorphes, e.g., rabbits. Animals of all ages are included, e.g. adults, animals of medium age and seniors.
Of primary interest with respect to the present invention are dogs and cats.

Plaque is a soft, gelatinous material composed of bacteria and their metabolic byproducts, oral debris, and salivary components. Mature plaque is not removed by normal actions of the tongue or by rising of the mouth. Rather, mechanical abrasion from chewing or tooth brushing is necessary for plaque removal.

Left undisturbed, aerobic and facultative anaerobic bacteria proliferate as the plaque thickens and matures. Over time, salivary calcium salts are deposited on the plaque, producing calculus. Calculus is a hard deposit that provides a rough surface, promoting accumulation of more plaque and also contributing to tissue damage as it extends into the gingival sulcus. Gingivitis occurs when plaque and calculus form at the neck of the tooth, leading to inflammation and tissue damage. As the gingival sulcus enlarges into a periodontal pocket, the area provides an oxygen-depleted environment that allows proliferation of anaerobic bacteria. Periodontal disease becomes established when the periodontal ligament is exposed to plaque, bacteria, and bacterial byproducts.

In some animals gingivitis persists without progressing into periodontitis. However, in most, untreated gingivitis eventually progresses to periodontal disease. Clinical signs of gingivitis and periodontal disease include oral malodor, gingival sensitivity and bleeding, tooth loss, and difficulty in eating.

The presence and proliferation of certain species of anaerobic bacteria and the inflammatory responses of the host contribute to the progressive destruction of the periodontium. As the supporting connective tissues and adjacent bone are weakened, teeth become loose and may be lost. Periodontal disease itself causes discomfort and pain and, if left untreated, can lead to bacteremia.

Odontoclastic resorptive lesions in cats also have been associated with gingival inflammation and, possibly, periodontal disease.

Since the majority of periodontal pathogens are gram-negative bacteria which release endotoxins (lipopolysaccharides - LPS) dental diseases are a potential risk factor for systemic disease in pets resulting in a systemic bacteremia or LPS challenge.

The importance of maintaining healthy periodontal tissues is of greater significance when considered that chronic periodontal disease my have a systemic as well as local effects. The most important factor that influences the development of gingivitis and periodontal disease in the dog and cat is the presence and persistence of undisturbed plaque on tooth surface. Once plaque has been deposited on the surface of the tooth, it may be reduced mechanically through abrasion provided by diet, chewing on supplemental chew toys or foods. The use of antimicrobial agents like chlorhexidine digluconate in conjunction with brushing, and the use of a chemical mouthwash is not effective in removing the hardened calculus that forms when plaque is allowed to accumulate.

Trisodium L-ascorbic acid-2-monophosphate is commercially available, e.g. from Roche Vitamins AG, Switzerland, under the tradename STAY-C^{®}50. Suitably, this salt is present in the pet food compositions of this invention in a concentration of from about 0.001 % by weight to about 5 % by weight, preferably in a concentration of from about 0.01 % by weight to about 3 % by weight, more preferably in a concentration of from about 0.1 % by weight to about 1 % by weight.

The polymer that may additionally be present in the per food compositions of this invention may be a homopolymer or a copolymer or mixtures thereof. Polymers capable of improving the uptake and retention of an ascorbic acid derivative in the oral cavity for use in the compositions of the present invention are natural or synthetic polymers which can be non-ionic, anionic, cationic or amphoteric. Natural polymers are preferred.
Examples of natural polymers for use in the present invention are polysaccharides such as carrageenan, guar gum, galactoarabinan and pectin, and derivatives thereof, especially cellullose derivatives such as hydroxyethylcellulose, hydroxypropylcellulose, hydroxypropylmethylcellulose, carboxymethyl cellulose and sodium carboxymethyl cellulose, quaternized hydroxyethylcellulose (Polymer JR 400, Polyquaternium 10), guar derivatives which are known as Jaguar types, and cellulose-based anionic polysaccharides such as celluloseacetatphthalat. Examples of synthetic polymers are non-ionic polymers such as polyvinylpyrrolidone and copolymers thereof with vinyl acetate; polyvinylalcohol, polyethyleneglycol und block copolymers of the ABA-type from polyethyleneglycol-polypropyleneglycol-polyethyleneglycol. Examples of anionic synthetic polymers are especially polyacrylic acid, polymethacrylic acid and their copolymers as well as copolymers of itaconic acid and styrene sulfonic acid.

Examples of cationic (or basic) polymers are copolymers, based on vinyl imidazol or dimethylaminoethyl methacrylamide (quaternized or non-quaternized). An amphoteric polymers is, e.g., Amphomer (National Starch).

More specifically, examples of polymers for use in the present invention are Guar gum;
- pectin;
- carboxymethylcellulose;
- quaternized hydroxyethylcellulose, e.g., Polyquaternium 10;
- carrageenan, e.g., carrageenan XP 3172;
- xanthan gum;
- acrylates/C₁₀₋₃₀ alkylacrylate crosspolymers as commercially available as Pemulen TR-1 and TR-2;
- Gantrez types, e.g., Gantrez S-97;
- polyvinyl pyrrolidone homopolymers as commercially available as PVP types;
- N-vinylpyrrolidone/acrylic acid copolymers as disclosed in EP 0 691 124 as AVP.

The polymer is suitably be used in an amount of from about to about 0.01 % by weight to about 5% by weight, preferably about 0.01 % by weight to about 3% by weight, based on the total weight of the pet food composition

The pet food according to the present invention may be based on any conventional pet food. There is a wide range of pet foods available which may be grouped into (a) complete diets, (b) complementary diets, and (c) snacks and treats. Complete diets may be fed in addition to water for an extended period as the sole source of nutrients and will provide for all the energetic and nutrient needs of the animal and the physiological state for which it is intended. Complementary diets normally are not sufficient to ensure that all nutrient and energy requirements are met unless fed in combination with another foodstuff or diet. Snacks and treats are appetizers or for occasional feeding and are considered as complementary products. There are, however, a number of products available intended to form part of the daily diet or playing a role in animal well-being, e.g. dental chews. In the present invention dental chews are especially suitable.

The pet food of the present invention may be in a dry, canned, semi-moist or baked form. Typical components of such compositions, in addition to Inventive Ingredients, are crude protein, crude fat, carbohydrates (NfE), starch, crude fibers, and ash, further on minerals, trace elements, vitamins, fatty acids, protein and amino acids, choline, carnitin, dietary fiber and substances required for balanced diets of the different animal species. Basic ingredients of such food compositions are
- Crude Protein including proteins and N-containing compounds of non-proteinaceous nature, e.g. acid amides, amines, free amino acids, ammonium salts, alkaloids;
- Crude Fat including neutral fats, lipoids (phospho-, sphingolipids, steroids) and other ethersoluble compounds;
- N-free Extractions (NFE) including polysaccharides (starch, glycogen), soluble saccharides (glucose, fructose, saccharose, lactose, maltose and oligosaccharides), and soluble fractions of cellulose, hemicellulose, lignin and pectins;
- Crude Fibers including insoluble fractions of cellulose, hemicellulose, lignin and other components of the cell wall like suberin, cutin etc.;
- Ash including minerals (macrominerals such as calcium, phosphorus, sodium, chloride, potassium, magnesium, and microminerals, i.e., trace elements, such as iron, copper manganese, zinc, iodine, selenium,) and further inorganic substances e.g. silicate.
- Vitamins including vitamins A, B₁, B₂, B₆, B₁₂, D, pantothenic acid, niacin, biotin, folic acid, linolic acid and choline.

Further components may, e.g. be omega-6-fatty acids, omega-3-fatty acids, L-carnitine, chondroitin sulfate, glucosamine, glutamine/glutamic acid, arginine, taurine and hydroxyproline.

Typical components which provide the ingredients for a dog food composition, in addition to Inventive Ingredients, comprise, e.g., chicken/beef/turkey, liver, broken pearl barley, ground corn, brute fat, whole dried egg, fowl protein hydrolyzate, vegetable oil, calcium carbonate, choline chloride, potassium chloride, iodinized salt, iron oxide, zinc oxide, copper sulfate, manganese oxide, sodium selenite, calcium iodate, provitamin D, vitamin B₁, niacin, calcium panthothenate, pyridoxin hydrochloride, riboflavin, folic acid, biotin, vitamin B₁₂.

Typical components which provide the ingredients for a cat food composition, in addition to Inventive Ingredients, comprise beef, chicken meat, dried chicken liver, lamb meat, lamb liver, pork, turkey meat, turkey liver, poultry meal, fish meal, fowl protein hydrolysate, animal fats, plant oils, soy bean meal, pea bran, maize gluten, whole dry egg, ground corn, corn flour, rice, rice flour, dry sugar beet molasses, fructooligosaccharides, soluble fibres, plant gums, cellulose powder, clay, bakers yeast, iodized sodium chloride, calcium sulfate, sodium triphosphate, dicalcium phosphate, calcium carbonate, potassium chloride, choline chloride, magnesium oxide, zinc oxide, iron oxide, copper sulfate, iron sulfate, manganese oxide, calcium jodate, sodium selenite, provitamin D, thiamine, niacin, calcium pantothenate, pyridoxine hydrochloride, riboffavin, folic acid, biotin, vitamin B₁₂, taurin, L-carnitine, caseine, D-methionine.

Wet pet food contains between about 70 and about 85 % moisture and about 15 and about 25 % dry matter.

A typical wet food for adult dogs may, e.g. comprise, in addition to Inventive Ingredients, at minimum 24 % protein, 15 % fat, 52 % starch, 0.8 % fibre, 3 % linolic acid, 0.6 % calcium, 0.5 % phosphorus, the Ca:P ratio being 1:1, 0.2 % potassium, 0.6 % sodium, 0.09 % chloride, 0.09 % magnesium, 170 mg/kg of iron, 15 mg/kg of copper, 70 mg/kg of manganese, 220 mg/kg of zinc, 4 mg/kg of iodine, 0.43 mg/kg of selenium, 74000 IU/kg of vitamin A, 1200 IU/kg of vitamin D,11 mg/kg of vitamin B₁, 6 mg/kg of riboffavin, 30 mg/kg of pantothenic acid, 20 mg/kg of niacin, 4.3 mg/kg of pyridoxine, 0.9 mg/kg of folic acid, 0.2 µg/kg of vitamin B₁₂, 2500 mg/kg of choline, 0.8 mg/kg biotin, 2500 mg/kg cholin, all percentages being based on dry weight of the total food composition.

A typical wet food for adult cats may, e.g. comprise, in addition to Inventive Ingredients, at minimum 44 % protein, 25 % fat, 20 % starch, 2.5 % fibre, 0.8 % calcium, 0.6 % phosphorus, 0.8 % potassium, 0.3 % sodium, 0.09 % chloride, 0.08 % magnesium, 0.25 % taurin, 170 mg/kg of iron, 15 mg/kg of copper, 70 mg/kg of manganese, 220 mg/kg of zinc, 4 mg/kg of iodine, 0.43 mg/kg of selenium, 74000 IU/kg of vitamin A, 1200 IU/kg of vitamin D, 11 mg/kg of vitamin B₁, 6 mg/kg of riboflavin, 30 mg/kg of pantothenic acid, 20 mg/kg of niacin, 4.3 mg/kg of pyridoxine, 0.9 mg/kg of folic acid, 0.2 µg/kg of vitamin B₁₂, 2500 mg/kg of choline, 0.8 mg/kg biotin, 2500 mg/kg cholin, all percentages being based on dry weight of the total food composition.

Dry pet food contains between about 6 and about 14 % moisture and about 86 % or more dry matter.

A typical dry food for adult dogs may, e.g. comprise, in addition to Inventive Ingredients, at minimum 25 % protein, 12 % fat, 41.5 % starch, 2.5 % fibre, 1 % linolic acid, 1 % calcium, 0.8 % phosphorus, the Ca:P ratio being 1:1, 0.6 % potassium, 0.35 % sodium, 0.09 % chloride, 0.1 % magnesium, 170 mg/kg of iron, 35 mg/kg of copper, 70 mg/kg of manganese, 220 mg/kg of zinc, 4 mg/kg of iodine, 0.43 mg/kg of selenium, 15000 IU/kg of vitamin A, 1200 IU/kg of vitamin D,11 mg/kg of vitamin B₁, 6 mg/kg of riboflavin, 30 mg/kg of pantothenic acid, 20 mg/kg of niacin, 4.3 mg/kg of pyridoxine, 0.9 mg/kg of folic acid, 0.2 µg/kg of vitamin B₁₂, 2500 mg/kg of choline, 0.8 mg/kg biotin, all percentages being based on dry weight of the total food composition.

A typical food for adult cats may, e.g. comprise, in addition to Inventive Ingredients, at minimum 32 % protein, 15 % fat, 27.5 % starch, 11 % dietetic fibres, 4.5 % fibre, 3.4 % linolic acid, 0.08 % arachionic acid, 0.15 % taurin, 50 mg/kg L-carnitin, omega 6/3 = 5, 1 % calcium, 0.8 % phosphorus, the Ca:P ratio being at least 1:1, 0.6 % potassium, 0.4 % sodium, 0.6 % chloride, 0.08 % magnesium, 190 mg/kg of iron, 30 mg/kg of copper, 60 mg/kg of manganese, 205 mg/kg of zinc, 2.5 mg/kg of iodine, 0.2 mg/kg of selenium, 25000 IU/kg of vitamin A, 1500 IU/kg of vitamin D, 20 mg/kg of vitamin B₁, 40 mg/kg of riboflavin, 56 mg/kg of pantothenic acid, 153 mg/kg of niacin, 14 mg/kg of pyridoxine, 3.2 mg/kg of folic acid, 0.2 mg/kg of vitamin B₁₂, 1 mg/kg of biotin, 3000 mg/kg of choline, all percentages being based on dry weight of the total food composition.

A typical dry food for adult dogs with teeth problems comprises, in addition to Inventive Ingredients, chicken by-product meal, corn meal, brewers rice, powdered cellulose, soybean mill run, animal fat (preserved with BHA, propyl gallate and citric acid), dried egg product, vegetable oil, natural flavor, flaxseed, taurine, preserved with BHT and BHA, beta-carotene, minerals (potassium chloride, salt, ferrous sulfate, zinc oxide, copper sulfate, manganous oxide, calcium iodate, sodium selenite), vitamins (choline chloride, vitamin A supplement, vitamin D₃ supplement, vitamin E supplement, niacin, thiamine, calcium pantothenate, pyridoxine hydrochloride, riboflavin, folic acid, biotin, vitamin B₁₂ supplement).

A typical dry food for adult cats with teeth problems comprises, in addition to Inventive Ingredients, chicken by-product meal, brewers rice, corn gluten meal, animal fat (preserved with BHA, propyl gallate and citric acid), corn meal, powdered cellulose, chicken liver flavor, vegetable oil, DL-methionine, taurine, preserved with BHT and BHA, β-carotene, minerals (potassium chloride, calcium carbonate, salt, calcium sulfate, ferrous sulfate, zinc oxide, copper sulfate, manganous oxide, calcium iodate, sodium selenite), vitamins (choline chloride, vitamin A supplement, vitamin D₃ supplement, niacin, thiamine, calcium pantothenate, riboflavin, pyridoxine hydrochloride, folic acid, biotin, vitamin B₁₂ supplement).

Typical components which provide the ingredients for a dog treat composition, e.g. cake or biscuit, may comprise vegetable by-products (wheat meal), meat and animal by-products (meat meal), grain (wheat), minerals, oils and fat (plant and animal fats).

A typical dog treat may, e.g. comprise at minimum 10 % water, 24 % protein, 6 % fat, 27.5 % starch, 6.5 % ash, 2.5 % fibre, 1.2 % calcium, 0.8 % phosphorus and 0.25 % sodium.

Typical components which provide the ingredients for a cat treat composition, e.g. cake or biscuit, may comprise vegetable products, meat and animal by-products, grain (wheat), minerals and vegetable by-products.

A typical cat treat may, e.g. comprise, in addition to Inventive Ingredients, at minimum 12 % water, 10 % protein, 2.5 % fat, 6 % ash, 2 % fibre.

A preferred pet food composition of the present invention is in the form of a treat.

Inventive Ingredients may be incorporated into conventional pet food e.g., into dry pet food by spraying an aqueous solution containing one or more Inventive Ingredients on the food composition while thoroughly mixing the composition, or by adding one or more Inventive Ingredients to the dough. Inventive Ingredients may be added simultaneously, e.g. at the same time and even as a premix, or consecutively as single Inventive Ingredient at a time or as a premix. Premixes may also include one or more of the other components of the final composition.

Dry food may be prepared, e.g., by screw extrusion including cooking, shaping and cutting of raw ingredients into a specific kibble shape and size in a very short period of time, while simultaneously destroying detrimental micro-organisms. The ingredients may be mixed into homogenous expandable dough and cooked in an extruder (steam/pressure) and forced through a plate under pressure and high heat. After cooking, the kibbles are then allowed to cool, before optionally being sprayed with a coating which may include liquid fat or digest including liquid or powdered hydrolyzed forms of an animal tissue such as liver or intestine from, e.g., chicken or rabbit. Hot air drying then reduces the total moisture content to 10 % or less.

Canned (wet) food may be prepared, e.g., by blending the raw ingredients including meats and vegetables, fatty acids, gelling agents, gravies, vitamins, minerals and water.

The mix is then fed into cans on a production line, the lids are sealed on and the filled cans are sterilized at a temperature of about 130°C for about 50 to 100 min.

The present invention provides the use of the composition of the invention in preventing or treating calculus, plaque, gingivitis and periodontal disease in pets.

The effect of the composition of the invention may be determined by various assays. The amount of plaque forming bacteria may be determined by counting the bacteria commonly considered responsible for the pathogenesis of periodontitis, e.g. *Streptococcus mutans, Eikenella corrodens* and *Porphyromonas gingivalis,* and further *Prevotella ssp, Bacteroides gingivalis, Bacteroides intermedius, Actinobacillus* and *Actinobacillus.* For example, subgingival plaque may be taken from the maxillary premolars of dogs with a scalar. Saliva, tongue dorsum and bucal mucosa may be obtained as cotton swabs. Each specimen may be placed immediately, e.g., in sterile oxygen free tubes containing, e.g. 10 ml GAM broth and stored in an anaerobic glove box with 70 % N₂,15% CO_{2,} and 15% H₂. The diluted suspension of each sample may, e.g., be placed on Brain Heart Infusion agar with 7% horse blood for total counts, and Brucella HK agar with 7% horse blood for counts of genus *Porphyromonas* as black-pigmented bacteria. Identification and bacterial count of representative colonies may be carried out using Rapid ANA II system, AIP 20A and API-ZYM systems. For *Streptococcus mutans* a Brain Heart Infusion would be used, too. The conditions used would be aerobic. After incubation at 37°C for 48 hours, MICs would be estimated using the systems mentioned above.

Gingivitis (oral inflammation) may be determined by measuring the gingival index, a method for estimating severity of inflammation of the gums.

To determine changes in, especially, white blood cells, a total blood count may be carried out.
- Red blood cell count: Hemoglobin, hematocrit, number of erythrocytes, erythrocyte indices MCHC, MCH, MCV.
- White blood cell count: Total number of leukocytes, differential blood count (Basophiles, neutrophiles, eosinophiles, monocytes, thrombocytes, B - and T-lymphocytes) relative and absolute.

The cellular profile may be used to determine changes in the humoral immune system. Therefore IgA and IgG concentration may be estimated.

Parameters for the determination of the antioxidant capacity include TEAC (Trolox equivalent antioxidant capacity) which may be measured, e.g. after a 1:1 dilution with a spctrophotometer, on which absorbance values were recorded over 3 minutes, according to the method of Armstrong and Browne, Adv Exp Med Biol 366:43-58 (1994), Total Antioxidant Capacity (TAC), Erythrocyte Superoxid Dismutase (SOD) which may, e.g., be determined based on the method developed by McCord and Fridovich, J Biol Chem 244:6056-63 (1969) coupling O₂-generators (xanthine and xanthine oxidase (XOD)) with an O₂-detector [2-(4-iodophenyl)-3-(4-nitrophenol)-5-phenyltetrazolium chloride] and monitoring absorbance in a spectrophotometer; Ferritin which may be determined by an enzyme-linked immunoassay, Ceruloplasmin which may be determined by a colorimetric method to determine ceruloplasmin oxidase activity; Vitamin E and C; CK; GOT which may be determined by using a fluorescence detector at a wavelength of 334 nm.

The effect of STAY-C^{®}50 will now be illustrated in more detail by the following experimental results. These results are described with reference to the drawing. In the drawing,
Figure 1 shows a graph representing the gingivitis indices based on control and treatment diet,
Figure 2 shows a graph representing the plaque indices based on control and treatment diet,
Figure 3 shows a graph representing the calculus indices based on control and treatment diet, and
Figure 4 shows a graph representing the antioxidative capacity based on control and treatment diet.

### Experiments:

18 domestic shorthair (DSH) breed cats (mean age 3.83±1.85 years, mean body weight 4.2 +1.1 kg) were divided by age and gender into two equal groups (n=9) to be fed for 28 days with a
- control diet or
- treatment diet (2.3 g STAY-C^{®} 50/kg cat food)

As the "clean tooth" model was used, all cats received a dental cleaning consisting of ultrasonic scaling and, where necessary, using an abrasor after Gracey, before the beginning of the study. The animals were then treated with the diets for 28 days. The analysis of the different indices were carried out according known methods.

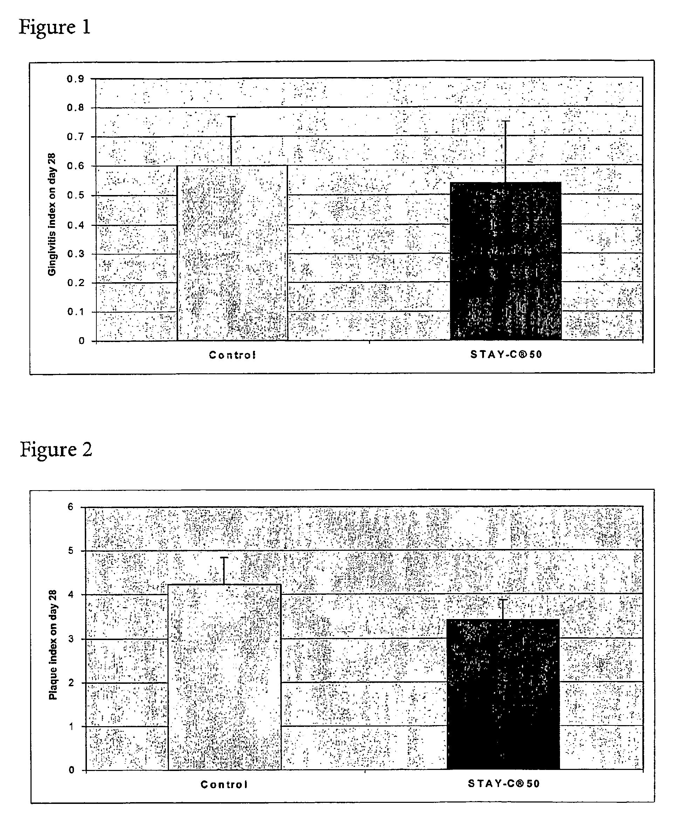

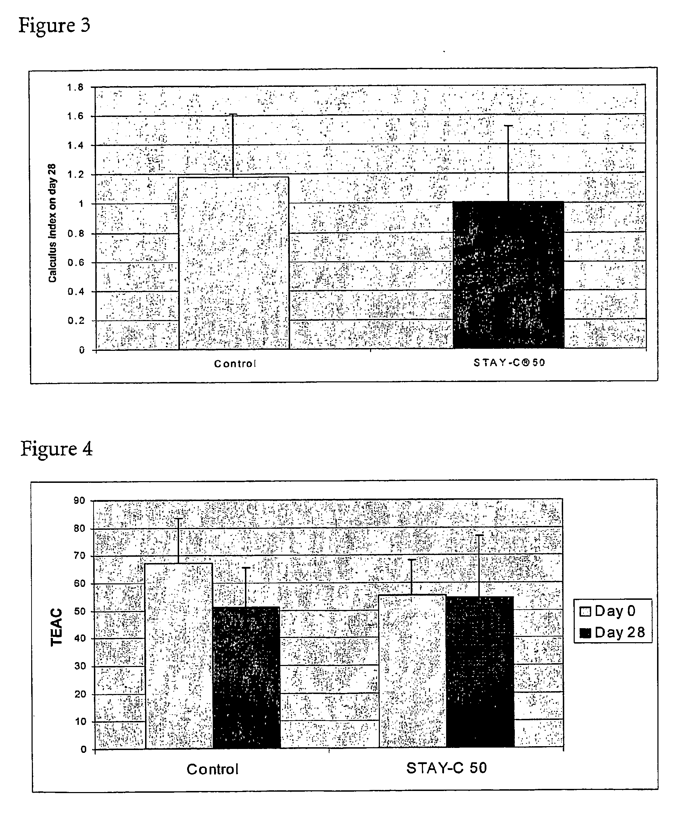

The results, which are partially shown in the figures 1 to 4 are as follows:
- No changes in systemic health, body weight, blood counts or serum biochemistry where observed in any of the two groups.
- Gingivitis indices in the treatment group where not increased compared to a 28% increase in control group.
- Plaque indices were reduced significantly in the treatment group.
- Calculus indices were reduced about 14% compared to control group.

The following examples illustrate the invention further.

### Example 1

To commercial dry dog food (Hill's Science diet "Canine Maintenance dry" for dogs as supplied by Hill's Pet Nutrition GmbH, Liebigstrasse 2-20, D- 22113) trisodium L-ascorbic acid-2-monophosphate (STAY-C^{®}50 as supplied by Roche Vitamins, Basel) is added in an amount sufficient to provide a concentration of 4500 mg per kg in the final food composition before extruding the entire blend. The food composition is dried to contain dry matter of about 90 % by weight.

### Example 2

Commercial dry dog food (Hill's Science diet "Canine Maintenance dry" for dogs as supplied by Hill's Pet Nutrition GmbH, Liebigstrasse 2-20, D- 22113) is sprayed with an aqueous solution of STAY-C^{®}50 and in an amount sufficient to provide 0.01 to 3.0 % by weight of STAY-C^{®}50. Further E and β-carotene are incorporated in an amount sufficient to provide 300 IU vitamin E/kg and 280 mg β-carotene/kg in the final food composition before extruding the entire dye. The food composition is dried to contain dry matter of about 90 % by weight.

### Example 3

Commercial wet dog food (Hill's Science diet "Canine Maintenance wet" for dogs as supplied by Hill's Pet Nutrition GmbH, Liebigstrasse 2-20, 22113 Hamburg, Germany) is mixed with an aqueous solution of STAY-C^{®}50 in an amount sufficient to provide 0.01 to 3.0 % by weight of STAY-C^{®}50 in the final food composition. Further Vitamin E and β-carotene are incorporated in an amount sufficient to provide 300 IU vitamin E/kg and 280 mg β-carotene/kg in the final food composition before cooking the entire blend. The food composition is dried to contain a dry matter of about 90 % by weight.

### Example 4

Commercial dog treats (Mera Dog "Biscuit" for dogs as supplied by Mera Tiernahrung GmbH, Marienstrasse 80-84, 47625 Kevelaer-Wetten, Germany) are sprayed with an aqueous solution of STAY-C^{®}50 in an amount sufficient to provide 0.01 to 3.0 % by weight of STAY-C^{®}50 in the final food composition. Further Vitamin E and β-carotene are incorporated in an amount sufficient to provide 300 IU vitamin E/kg and 280 mg β-carotene/kg in the final food composition before cooking the entire blend. The food composition is dried to contain a dry matter of about 90 % by weight.

### Example 5

Commercial dry cat food (Hill's Science diet "Feline Maintenance dry" for cats as supplied by Hill's Pet Nutrition GmbH, Liebigstrasse 2-20, D- 22113) is sprayed with an aqueous solution of STAY-C^{®}50 in an amount sufficient to provide 0.01 to 3.0 % by weight of STAY-C^{®}50 in the final food composition. Further Vitamin E and β-carotene are incorporated in an amount sufficient to provide 300 IU vitamin E/kg and 280 mg β-carotene/kg in the final food composition before cooking the entire blend. The food composition is dried to contain a dry matter of about 90 % by weight.

### Example 6

Commercial wet cat food (Hill's Science diet "Feline Maintenance wet" for cats as supplied by Hill's Pet Nutrition GmbH, Liebigstrasse 2-20, D- 22113) is sprayed with an aqueous solution of STAY-C^{®}50 in an amount sufficient to provide 0.01 to 3.0 % by weight of STAY-C^{®}50 in the final food composition. Further Vitamin E and β-carotene are incorporated in an amount sufficient to provide 300 IU vitamin E/kg and 280 mg β-carotene/kg in the final food composition before cooking the entire blend. The food composition is dried to contain a dry matter of about 90 % by weight.

### Example 7

Commercial cat treats (Whiskas Dentabits for cats as supplied by Whiskas, Masterfoods GmbH, Eitzer Str. 215, 27283 Verden/Aller, Germany) are sprayed with an aqueous solution of STAY-C^{®}50 in an amount sufficient to provide 0.+01 to 3.0 by weight of STAY-C^{®}50 in the final food composition. Further Vitamin E and β-carotene are incorporated in an amount sufficient to provide 300 IU vitamin E/kg and 280 mg β-carotene/kg in the final food composition before cooking the entire blend. The food composition is dried to contain a dry matter of about 90 % by weight.

## Claims

1. Trisodium L-ascorbic acid-2-monophosphate for use in a method for preventing or treating calculus, plaque, gingivitis and periodontal disease in pets, wherein trisodium L-ascorbic acid-2-monophosphate is added to pet food in an amount to provide a concentration of 0.01 % by weight to 5% by weight.

2. Use according to claim 1, wherein trisodium L-ascorbic acid-2-monophosphate is used in combination with a polymer for improving the uptake and retention of trisodium L-ascorbic-2-monophosphate.

3. Use according to any claim 1 or 2, wherein the polymer is selected from the group consisting of
- polysaccharides such as carrageenan, guar gum, galactoarabinan and pectin;
- cellulllose derivatives such as hydroxyethylcellulose, hydroxypropylcellulose, hydroxypropylmethylcellulose, carboxymethyl cellulose, sodium carboxymethyl cellulose, quaternized hydroxyethylcellulose;
- cellulose-based anionic polysaccharides such as celluloseacetatphthalat;
- non-ionic polymers such as polyvinylpyrrolidone, polyvinylalcohol, polyethyleneglycol, polyethyleneglycol-polypropyleneglycol-polyethyleneglycol; and
- anionic synthetic polymers as polyacrylic acid, polymethacrylic acid.

## Patentansprüche

1. Trinatrium-L-ascorbinsäure-2-monophosphat für die Verwendung in einem Verfahren zur Vorbeugung oder Behandlung von Calculus, Plaque, Gingivitis und periodontalen Erkrankungen bei Haustieren, wobei Trinatrium-L-ascorbinsäure-2-monophosphat dem Haustierfutter in einer Menge zugesetzt wird, um eine Konzentration von 0,01 Gew.-% bis 5 Gew.-% bereitzustellen.

2. Verwendung gemäß Anspruch 1, wobei Trinatrium-L-ascorbinsäure-2-monophosphat in Kombination mit einem Polymer verwendet wird zur Verbesserung der Aufnahme und Retention von Trinatrium-L-ascorbinsäure-2-monophosphat.

3. Verwendung gemäß einem der Ansprüche 1 oder 2, wobei das Polymer aus der Gruppe gewählt ist, die aus Folgendem besteht:
- Polysacchariden, wie Carrageenan, Guargummi, Galaktoarabinan und Pektin;
- Cellulosederivaten, wie Hydroxyethylcellulose, Hydroxypropylcellulose, Hydroxypropylmethylcellulose, Carboxymethylcellulose, Natriumcarboxymethylcellulose, quaternisierter Hydroxyethylcellulose;
- anionischen Polysacchariden auf Cellulosebasis, wie Celluloseacetatphthalat;
- nichtionischen Polymeren, wie Polyvinylpyrrolidon, Polyvinylalkohol, Polyethylenglykol, Polyethylenglykol-Polypropylenglykol-Polyethylenglykol; und
- anionischen synthetischen Polymeren wie Polyacrylsäure, Polymethacrylsäure.

## Revendications

1. Acide L-ascorbique-2-monophosphate trisodique destiné à être utilisé dans une méthode de prévention ou de traitement du tartre, de la plaque, de la gingivite et d'une maladie parodontale chez les animaux familiers, l'acide L-ascorbique-2-monophosphate trisodique étant ajouté à l'aliment pour animaux dans une quantité pour donner une concentration de 0,01 % en poids à 5 % en poids.

2. Utilisation selon la revendication 1, **caractérisée en ce que** l'acide L-ascorbique-2-monophosphate trisodique est utilisé en combinaison avec un polymère pour améliorer l'absorption et la rétention de l'acide L-ascorbique-2-monophosphate trisodique.

3. Utilisation selon l'une ou l'autre des revendications 1 et 2, **caractérisée en ce que** le polymère est choisi dans le groupe constitué de
- polysaccharides tels que le carraghénane, la gomme de guar, le galactoarabinane et la pectine ;
- dérivés de cellulose tels que l'hydroxyéthylcellulose, l'hydroxypropylcellulose, l'hydroxypropylméthylcellulose, la carboxyméthylcellulose, la carboxyméthylcellulose sodique, l'hydroxyéthylcellulose quaternisée ;
- polysaccharides anioniques à base de cellulose tels que l'acétate phtalate de cellulose ;
- polymères non ioniques tels que la polyvinylpyrrolidone, l'alcool polyvinylique, le polyéthylèneglycol, le polyéthylèneglycolpolypropylèneglycol-polyéthylèneglycol ; et
- polymères anioniques de synthèse tels que l'acide polyacrylique, l'acide polyméthacrylique.
